# EUROPEAN PATENT APPLICATION

(11) **EP 3 173 070 A1**
(43) Date of publication of application: **31.05.2017**
(21) Application number: 16200612.6
(22) Date of filing: 25.11.2016
(51) Int. Cl.: A61K 9/00, A61K 9/19

(54) **LYOPHILIZED COMPOSITION OF BENZIMIDAZOLE COMPOUND**

(30) Priority: 26.11.2015 TR 201515019
(71) Applicant: Verano Ilac Sanayi Ve Ticaret A.S., Istanbul (TR)
(72) Inventor: TÜRKYILMAZ, Ali, 34460 Istanbul (TR); YELKEN, Gülay, 34460 Istanbul (TR)
(74) Representative: Sevinç, Erkan

(57) **Abstract**

The present invention relates to a lyophilized composition comprising a substituted or unsubstituted benzimidazole compound.

## Description

### Field of Invention

The present invention relates to a lyophilized composition comprising a substituted or unsubstituted benzimidazole compound.

### Background of Invention

Antiparasitics are a class of medications which are indicated for the treatment of parasitic diseases such as those caused by endoparasites such as nematodes (roundworms), cestodes (tapeworms), trematodes (flatworms), and parasitic protozoa (the cause of malaria).

Anthelmintics are a group of antiparasitic drugs that expel parasitic worms (helminths) and other internal parasites from the body by either stunning or killing them and without causing significant damage to the host. They may also be called vermifuges (those that stun) or vermicides (those that kill). They are used to treat people or animals who are infected by helminths, a condition called helminthiasis.

Benzimidazole is a heterocyclic aromatic organic compound. This bicyclic compound consists of the fusion of benzene and imidazole.

Bezimidazoles are mostly used on livestock like cattle, sheep, goats, etc. Besides that, use on dogs and cats is available. It is available in the form of injectables and drenches or tablets, pills, etc.

Ricobendazole is a methylcarbamate benzimidazole with a broad-spectrum anthelmintic activity. Ricobendazole is a key metabolite of albendazole. Ricobendazole is a veteran anthelmintic (wormer) compound belonging to the chemical class of the benzimidazoles.

Chemical name of ricobendazole is Methyl [5-(propane-1-sulfinyl)-1 H-benzoimidazol-2-yl]-carbamate and structure is illustrated with Formula I given below.

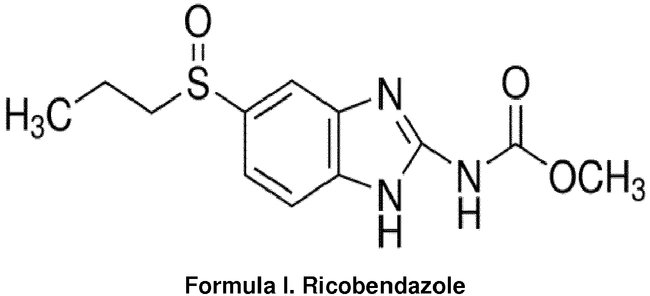

In prior art, albendazole derivatives and the use for the treatment of helminthiasis first disclosed in US3915986A. US4076828A discloses the parenteral administration of benzimidazole derivatives in treating helminthiasis. EP705101B1 discloses the oral administration of benzimidazole derivatives and the use as an anti-parasitic agent.

A problem found in the prior art with existing anthelmintic compounds including benzimidazole anthelmintics, is the need to deliver the compound by injection due to the particularly poor solubility. In addition, poor solubility of this type of compound in aqueous environments. As the solubility of the anthelmintic is often poor, injections are not readily absorbed and often may cause pain to the animal or human patient due to inadequate absorption or precipitate formation.

A further problem noted with prior art formulations is pain, swelling and inflammation at the injection site. These reactions may also be associated with the anthelmintic compound such as a reaction from the subject against precipitated drug.

In this invention, to fulfill requirements of the injectable composition of benzimidazoles, a lyophilized composition have been developed comprising a substituted or unsubstituted benzimidazole compound.

### Description of Invention

The present invention relates to a lyophilized composition comprising a substituted or unsubstituted benzimidazole compound.

Benzimidazoles are characterised by having poor solubility/dispersion characteristics in an aqueous environment. It is understood by the inventors that this poor solubility/dispersion may result in corresponding poor absorption, which may lead to poor pharmacokinetics. If such a compound is administered absent of a suitably formulated delivery system, the compound may either not be absorbed or be only poorly absorbed within an aqueous environment such as the blood stream.

The main object of the present invention is to enhance solubility of a substituted or unsubstituted benzimidazole compound in aqueous environments.

According to the object of the present invention there is provided the use of a formulation substantially as described above in the manufacture of a medicament to increase the solubility/dispersion of a substituted or unsubstituted benzimidazole compound wherein the substituted or unsubstituted benzimidazole compound is characterised by having poor solubility/dispersion characteristics in an aqueous environment.

In an embodiment, the benzimidazole compound is selected from albendazole, ricobendazole, flubendazole, fenbendazole, mebendazole, oxfenbendazole, parbendazole, thiabendazole, triciabendazole or combinations, analogues, derivatives thereof.

In a preferred embodiment, the benzimidazole compound is preferably ricobendazole. According to an embodiment of the present invention, the lyophilized composition contains ricobendazole in an amount of 0.5% to 90% of total weight of the pharmaceutical formulation.

In another embodiment, the lyophilized composition is for injectable administration.

According to this embodiment, the lyophilized composition is administered by intravenous, subcutaneously, intramuscular, intradermal, intracutaneous, intraruminal.

Ricobendazole is the injectable benzimidazole. Chemically ricobendazole is albendazole sulfoxide, the major metabolite of albendazole. Benzimidazoles are very poorly soluble in water, and therefore cannot be injected, because they get stuck at the injection site. They have to be delivered orally (in drenches, boluses, tablets, etc.). Ricobendazole is significantly more soluble in water than albendazole and can be injected.

Ricobendazole is an anti-parasitic nitroimidazole compound administered by injection, unlike most nitroimidazoles. Ricobendazole binds the colchicine site of tubulin, inhibiting microtubule polymerization. Ricobendazole also exhibits anticancer activity, potentiating the effects of taxanes and inhibiting cell proliferation in breast cancer cells, non-small cell lung cancer (NSCLC) cells, and melanoma cells.

The lyophilized composition of this invention comprises one or more pharmaceutically acceptable excipient which is selected from diluent/carrier, buffering agent, tonicity agent, pH adjuster, antimicrobial, wetting agent, solvent or mixtures thereof.

In one embodiment, the lyophilized composition comprising diluent/carrier is selected from mannitol, serum albumin (BSA), dextran, ficoll, gelatin, polyvinylpyrrolidone; dextrose, hydroxypropyl-β-cyclodextrin, lactose, mannitol, raffinose, sorbitol, sucrose, trehalose; β-alanine, glycine, histidine or mixtures thereof.

In one embodiment, the lyophilized composition comprising buffering agent is selected from bovine potassium acetate, sodium acetate, calcium chloride, citric acid, potassium citrate, sodium citrate, HEPES (4-(2-hydroxyethyl)-1-piperazineethanesulfonic acid), sodium bicarbonate, potassium dihydrogen phosphate, dipotassium phosphate, sodium dihydrogen phosphate, tris base, tris acetate, zinc chloride or mixtures thereof.

In one embodiment, the lyophilized composition comprising tonicity agent is selected from dextrose, glycerin, hydroxpropyl betadex, mannitol, potassium chloride, sodium chloride or mixtures thereof.

In one embodiment, the lyophilized composition comprising pH adjusters which is an acid and a base.

According to this embodiment, the lyophilized composition comprising an acid as a pH adjuster is selected from citric acid, tartaric acid, ascorbic acid, fumaric acid, malic acid, acetic acid, acid salts (amino acid hydrochlorides, sodium dihydrogen citrate, disodium hydrogen citrate, sodium acid phosphate), hydrochloric acid, nicotinic acid, acetylsalicylic acid, adipic acid or mixtures thereof.

In another embodiment, the lyophilized composition comprising a base as a pH adjuster is selected from sodium bicarbonate, sodium carbonate, sodium hydroxide, potassium carbonate, potassium carbonate, calcium carbonate, amino acid derivatives, alkali metal carbonate (such as sodium glycine carbonate) or mixtures thereof.

In an embodiment, the lyophilized composition comprising an antimicrobial is selected from benzyl alcohol, benzoic acid, boric acid, sorbic acid and their salts thereof, benzalkonium chloride, parahydroxybenzoic acids and their alkyl esters, methyl paraben, propyl paraben or mixtures thereof.

In one embodiment, the lyophilized composition comprising wetting agent is selected from benzalkonium chloride, benzethonium chloride, cetylpyridinium chloride, docusate sodium, glycine, hypromellose, phospholipids, poloxamer, polyoxyethylene alkyl ethers, polyoxyethylene castor oil derivatives, polyoxyethylene sorbitan fatty acid esters, polyoxyethylene stearates, sodium lauryl sulfate, sorbitan esters, tricaprylin or mixtures thereof.

In one embodiment, the lyophilized composition comprising solvent is selected from corn oil (maize), cottonseed oil, dibutyl phthalate, diethyl phthalate, dimethyl ether, dimethyl phthalate, dimethyl sulfoxide, dimethylacetamide, ethyl acetate, ethyl alcohol, ethyl lactate, ethyl oleate, glycerin, glycofurol, isopropyl alcohol, isopropyl myristate, isopropyl palmitate, light mineral oil, medium-chain triglycerides, methyl lactate, mineral oil, monoethanolamine, octyldodecanol, olive oil, peanut oil, polyethylene glycol, polyoxyl 35 castor oil, propylene carbonate, propylene glycol, pyrrolidone, safflower oil, sesame oil, soybean oil, sunflower oil, triacetin, tricaprylin, triethanolamine, triethyl citrate, triolein, water, water-miscible or mixtures thereof.

In one embodiment, the lyophilized composition is used as anti-parasitic, anti-helmintic, anti-trematode, taenicide, nematicide.

In another embodiment, the lyophilized injectable composition is formulated for administration to human or animal.

Ricobendazole is used therapeutically in the treatment of helminthiasis and tapeworm infestations in man and animal. It prevents growth of parasites; reduce parasite numbers; kill parasites; kill incoming parasite larvae; lower the amount of incoming parasite larva and combinations thereof.

Ricobendazole is effective against gastrointestinal roundworms and lungworms of livestock including adults and L4-larvae of the most important species (e.g. of the genus *Bunostomum, Haemonchus, Ostertagia,Teladorsagia, Trichostrongylus, Cooperia,Nema todirus, Chabertia, Oesophagostomum, Trichuris, Dictyocaulus,* etc.) as well as arrested larvae of several species. It is also effective against most livestock tapeworms (e.g. *Moniezia)* and against adult liver flukes *(Fasciola hepatica* and *Fascioloides magna).* It is also effective against the major parasitic roundworms of dogs and cats *(e.g.Ancylostoma, Toxocara, Trichuris, Uncinaria*).

There are also a number of challenges surrounding the formulation and development of injectable forms such as solubility. Trace amounts of impurities from active pharmaceutical agents or excipients and the pH of the solution may cause the degradation of active pharmaceutical agent and this may cause a decrease in solubility when the compound is consistently introduced into aqueous environments.

The process for the preparation of the lyophilized composition comprising a substituted or unsubstituted benzimidazole compound comprises the following steps:
- all ingredients of the unit formula are mixed with the chosen solvent to obtain a solution/dispersion.
- pH of the obtained solution is adjusted, followed by filtration aseptically and filling into vials.
- lyophilization process is carried out under vacuum and sterilized by autoclave if needed.

It has been found that lyophilization enhances solubility of benzimidazole compound in the present injectable composition. In addition, precipitation and crystallization in the solution has been prevented.-The pharmaceutical formulation disclosed herein is a solution wherein benzimidazole compound such as ricobendazole, or pharmaceutically acceptable excipients are not precipitated.

### Example 1:

| **Ingredients** | **Amount (%)** |
|---|---|
| Ricobendazole | 0.5-90.0 |
| Dextrose | 0.5-90.0 |
| Sodium chloride | 0.1-5.0 |
| Hydrochloric acid* | q.s |
| Sodium hydroxide* | q.s |
| Water for injection | q.s |

| | |
|---|---|
| *for pH adjustment q.s.:quantity sufficient | |

### Example-2:

| **Ingredients** | **Amount (%)** |
|---|---|
| Ricobendazole | 0.5-90.0 |
| Mannitol | 0.5-90.0 |
| Benzyl alcohol | 0.1-5.0 |
| Sodium chloride | 0.1-5.0 |
| Hydrochloric acid* | q.s |
| Sodium hydroxide* | q.s |
| Water for injection | q.s |

| | |
|---|---|
| *for pH adjustment q.s.:quantity sufficient | |

### Example-3:

| **Ingredients** | **Amount (%)** |
|---|---|
| Ricobendazole | 0.5-90.0 |
| Sucrose | 0.5-90.0 |
| Polysorbate (Polyoxyethylene Sorbitan Fatty Acid Ester) | 0.1-20 |
| Benzyl alcohol | 0.1-5.0 |
| Sodium chloride | 0.1-5.0 |
| Hydrochloric acid* | q.s |
| Sodium hydroxide* | q.s |
| Water for injection | q.s |

| | |
|---|---|
| *for pH adjustment q.s.:quantity sufficient | |

### Example-4:

| **Ingredients** | **Amount (%)** |
|---|---|
| Ricobendazole | 0.5-90.0 |
| Mannitol | 0.5-90.0 |
| Polysorbat*(Polyoxyethylene Sorbitan Fatty Acid Esters)* | 0.01-20 |
| Methyl paraben | 0.01-2 |
| Propyl paraben | 0.01-2 |
| Sodium chloride | 0.1-5.0 |
| Hydrochloric acid* | q.s |
| Sodium hydroxide* | q.s |
| Water for injection | q.s |

| | |
|---|---|
| *for pH adjustment q.s.:quantity sufficient | |

### Production process of the examples above:

- all ingredients of the unit formula are mixed with the chosen solvent to obtain a solution/dispersion.
- pH of the obtained solution is adjusted, followed by filtration aseptically and filling into vials.
- lyophilization process is carried out under vacuum and sterilized by autoclave if needed.

## Claims

1. A lyophilized composition comprising a substituted or unsubstituted benzimidazole compound.

2. The lyophilized composition according to claim 1, wherein the benzimidazole compound is selected from albendazole, ricobendazole, flubendazole, fenbendazole, mebendazole, oxfenbendazole, parbendazole, thiabendazole, triciabendazole or combinations, analogues, derivatives thereof.

3. The lyophilized composition according to claim 2, wherein the benzimidazole compound is preferably ricobendazole.

4. The lyophilized composition according to claim 3, wherein ricobendazole in an amount of 0.5% to 90% of total weight of the pharmaceutical formulation.

5. The lyophilized composition according to claim 1 to 4, wherein the composition is for injectable administration.

6. The lyophilized composition according to claim 5, wherein the composition is administered by intravenous, subcutaneously, intramuscular, intradermal, intracutaneous, intraruminal.

7. The lyophilized composition according to any preceding claim, wherein the composition comprising one or more pharmaceutically acceptable excipient which is selected from diluent/carrier, buffering agent, tonicity agent, pH adjuster, antimicrobial, wetting agent, solvent or mixtures thereof.

8. The lyophilized composition according to any preceding claim, wherein the composition is used as anti-parasitic, anti-helmintic, anti-trematode, taenicide, nematicide.

9. The lyophilized composition according to any preceding claim, wherein the injectable composition is formulated for administration to human or animal.

10. The process for the preparation of the lyophilized composition comprising a substituted or unsubstituted benzimidazole compound according to any preceding claim, wherein the process comprises the following steps:
• all ingredients of the unit formula are mixed with the chosen solvent to obtain a solution/dispersion.
• pH of the obtained solution is adjusted, followed by filtration aseptically and filling into vials.
• lyophilization process is carried out under vacuum and sterilized by autoclave if needed.
